# EUROPEAN PATENT SPECIFICATION

(11) **EP 4 577 530 B1**
(45) Date of publication and mention of the grant of the patent: **09.12.2026**
(21) Application number: 23757947.9
(22) Date of filing: 22.08.2023
(51) Int. Cl.: C07D 319/12, C08J 11/12, C08J 11/02, C08L 67/04

(54) **PROCESS FOR SEPARATING MIXTURES OF LACTIDES AND GLYCOLIDE**
VERFAHREN ZUR TRENNUNG VON MISCHUNGEN AUS LACTIDEN UND GLYKOLID
PROCÉDÉ DE SÉPARATION DE MÉLANGES DE LACTIDES ET DE GLYCOLIDE

(30) Priority: 24.08.2022 EP 22191853
(43) Date of publication of application: 02.07.2025
(73) Proprietor: Purac Biochem B.V., 4206 AC Gorinchem (NL)
(72) Inventor: GOBIUS DU SART, Gerrit, 4206 AC GORINCHEM (NL); DOORNHEIM, Martin, 4206 AC GORINCHEM (NL)
(74) Representative: De Vries & Metman
(86) International application number: PCT/EP2023/073041
(87) International publication number: WO 2024/042084

(56) References cited:
- US-A1- 2015 065 732
- US-B1- 6 310 218

## Description

### TECHNICAL FIELD

The present invention is directed to a process for separating mixtures of lactide(s) and glycolide. More in particular, the present invention is directed to a process for recovering a lactide or glycolide from a mixture of lactide(s) and glycolide, by performing a melt crystallization at a suitable crystallization temperature, which is determined based on the crystallization point of the mixture.

### BACKGROUND

Mixtures of lactides and glycolide are commercially relevant due to the appearance of PLGA copolymers in the market. In the past two decades poly(lactic-co-glycolic acid) or PLGA copolymers have indeed been among the most attractive polymeric candidates used to fabricate devices for drug delivery and tissue engineering applications. PLGA is biocompatible and biodegradable, exhibits a wide range of erosion times, has tunable mechanical properties. In particular, PLGA has been extensively studied for the development of devices for controlled delivery of small molecule drugs, proteins, and other macromolecules in commercial use and in research. PLGA copolymers provide exiting opportunities for technical grade polyesters as well in view of their faster degradability at lower composting temperatures and improved barrier properties versus for instance polylactic acid (PLA).

PLGA is a copolymer of lactic acid and glycolic acid. PLGA is typically synthesized by means of ring-opening co-polymerization of two different monomers, the cyclic dimers of glycolic acid and lactic acid, respectively glycolide (i.e. 1,4-dioxane-2,5-diones) and lactide (3,6-Dimethyl-1,4-dioxan-2,5-dione). Polymers can be synthesized as either random or block copolymers thereby imparting additional polymer properties. During polymerization, successive monomeric units (of glycolic or lactic acid) are linked together in PLGA by ester linkages, thus yielding a linear, aliphatic polyester as a product. If so desired, other topologies are also possible, then requiring the use of multifunctional initiators (for star-shaped) or branching agents like bicyclic molecules, (poly)epoxides or radical chemistry.

In order to obtain lactide and glycolide, e.g. for preparing PLGA, it has been proposed in the art to synthesize or recover lactide or glycolide separately from their respective acids or polymers. For instance, EP 2 559 725 A1 reports a separate recovery of lactide from lactic acid or recycled PLA, or of glycolide from glycolic acid or recycled PGA. The disclosed process for the recovery of lactide from polylactide (PLA) or glycolide from polyglycolide (PGA) comprises a first step wherein PLA or PGA is brought into contact with a hydrolyzing medium and degraded hydrolytically to oligomers. In a further step, a cyclizing depolymerization of the oligomers obtained in the first step to the lactide or glycolide takes place.

US 2015/065732 describes a method for preparing lactide from recovered polylactic acid, wherein the recovered polylactic acid is pretreated, the treated polylactic acid is extruded to yield a polylactic acid melt, a chain scission reaction is carried out, and a depolymerization reaction is conduced to yield a crude lactide. The crude lactide is then purified and the purified lactide is crystallized.

However, in the prior art, also mixtures of lactides and glycolide may be prominently available, e.g. as side-products or product solution obtained during PLA, PGA or PLGA polymerizations. Such mixtures provide a suitable but so far unexploited starting material for obtaining lactide and/or glycolide.

### SUMMARY

The present invention aims to provide a process for recovering lactide or glycolide from mixtures comprising lactide(s) and glycolide. It is an aim of the present invention to provide a process for separating mixtures that can be applied to any mixture of lactide(s) and glycolide, i.e. to mixtures that are of various origins.

In one aspect, a process is provided for separating mixtures comprising lactide(s) and glycolide, wherein said process comprises the steps of:
a) providing a mixture comprising lactide(s) and glycolide, wherein said mixture is provided in the molten state, and preferably wherein the total combined amount of lactide(s) and glycolide in said mixture is at least 70.0 wt%, based on the total weight of the mixture,
b) determining the crystallization point of said mixture,
c) determining a desired amount of lactide or of glycolide to be recovered from said mixture,
d) selecting a suitable crystallization temperature (T_{c}) based on the desired amount of lactide or of glycolide to be recovered as determined in step c),
e) subjecting said mixture to a melt crystallization at said temperature T_{c}, thereby obtaining a mother liquid and a crystallized product , wherein said crystallized product comprises crystalized lactide or crystallized glycolide, and
f) recovering said crystallized product.

In preferred embodiments of a process of the invention, the total combined amount of lactide(s) and glycolide in said mixture is at least 75.0 wt%, or at least 80.0 wt%, or at least 85.0 wt%, or at least 90.0 wt%, or at least 95.0 wt%, based on the total weight of the mixture.

In certain preferred embodiments of a process of the invention, the crystallization point of the mixture is determined by establishing a phase diagram of glycolide and the lactide which is the predominant lactide in said mixture.

In certain preferred embodiments of a process of the invention, the crystallization temperature (T_{c}) is comprised in a range from 30 to 95 °C, or from 50 to 95°C, or from 50 to 80°C, or from 60 to 90°C.

In certain embodiments of a process of the invention it is preferred that the crystallized product recovered in step f) is further purified by melting said crystallized product to a molten product and supplying the molten product as a new mixture in step a) of the separation process of the invention, thereby recovering purified crystallized product and a new mother liquid from said recovered crystallized product.

Alternatively or additionally, in certain embodiments of a process of the invention, the mother liquid obtained in step e), is supplied as a new mixture in step a) in the separation process of the invention, thereby recovering additional amounts of crystallized product and a new mother liquid.

In accordance with certain embodiments of the present process, at least 50%, or at least 60%, or at least 70%, or at least 75%, or at least 80%, or at least 85%, or at least 90% of the amount of lactide or of glycolide comprised in the (initial) mixture is recovered as crystallized product.

In certain embodiments of the present process, melt crystallization comprises
- at least one layer crystallization, and preferably said layer crystallization comprises at least one falling film crystallization and/or at least one static crystallization, and/or
- at least one suspension crystallization.

The crystallized product and optionally the purified crystallized product, obtained by carrying out the process of the invention may be recovered by filtration, centrifugation, or another solid-liquid separation means, or any combinations thereof.

In certain embodiments, the present invention provides a process wherein a lactide is separated from a mixture of lactide(s) and glycolide. To that end, the invention provides a process for separating lactide from a mixture comprising lactide(s) and glycolide, wherein said process comprises the steps of:
a) providing a mixture comprising lactide(s) and glycolide, wherein said mixture is provided in the molten state, and preferably wherein the total combined amount of lactide(s) and glycolide in said mixture is at least 70.0 wt%, based on the total weight of the mixture,
b) determining the crystallization point of said mixture,
c) determining a desired amount of lactide to be recovered from said mixture,
d) selecting a suitable crystallization temperature (T_{c}) based on the desired amount of lactide to be recovered as determined in step c),
e) subjecting said mixture to a melt crystallization at said temperature T_{c}, preferably a crystallization temperature (T_{c}) comprised between 30°C and 95°C, or between 50°C and 95°C, or between 60°C and 90°C, thereby obtaining a mother liquid and crystallized lactide; and
f) recovering said crystallized lactide,
g) optionally further purifying the crystallized lactide recovered in step f) by melting said crystallized lactide to a molten product and supplying the molten product as a new mixture in step a), thereby recovering purified crystallized lactide and a new mother liquid from said recovered crystallized lactide, and
h) optionally supplying mother liquid(s) obtained in any one of the previous steps, as a new mixture in step a), thereby recovering additional amounts of crystallized lactide and a new mother liquid.

In certain embodiments of a process of the invention, the crystallized lactide comprises an amount (in wt%) of glycolide which is less than 0.5 times, such as less than 0.2 times, the amount (in wt%) of glycolide in the initial mixture. In certain embodiments of the present process, the purification factor for glycolide of the crystallized lactide is in the range of 2 to 20.

In certain other embodiments, the present invention provides a process wherein glycolide is separated from a mixture of lactide(s) and glycolide. To that end, the invention provides a process for separating glycolide from a mixture comprising lactide(s) and glycolide, wherein said process comprises the steps of:
a) providing a mixture comprising lactide(s) and glycolide, wherein said mixture is provided in the molten state, and preferably wherein the total combined amount of lactide(s) and glycolide in said mixture is at least 70.0 wt%, based on the total weight of the mixture,
b) determining the crystallization point of said mixture,
c) determining a desired amount of glycolide to be recovered from said mixture,
d) selecting a suitable crystallization temperature (T_{c}) based on the desired amount of glycolide to be recovered as determined in step c),
e) subjecting said mixture to a melt crystallization at said temperature T_{c}, and preferably a crystallization temperature (T_{c}) comprised between 50°C and 80°C, such as between 60°C and 80°C, thereby obtaining a mother liquid and a crystallized glycolide comprising crystals of glycolide; and
f) recovering said crystallized glycolide,
g) optionally further purifying the crystallized glycolide recovered in step f) by melting said crystallized glycolide to a molten product and supplying the molten product as a new mixture in step a), thereby recovering purified crystallized glycolide and a new mother liquid from said recovered crystallized glycolide, and
h) optionally supplying mother liquid(s) obtained in any one of the previous steps, as a new mixture in step a), thereby recovering additional amounts of crystallized glycolide and a new mother liquid.

In certain embodiments of the present process, the crystallized glycolide comprises an amount (in wt%) of lactide(s) which is less than 0.5 times, such as less than 0.2 times, the amount (in wt%) of lactide(s) in the (initial) mixture. In certain embodiments of the present process, the purification factor for lactide of the crystallized glycolide is in the range of 2 to 20.

The present process allows to obtain separate glycolide or a lactide, such as L-lactide, out of mixtures comprising these components. Preferably, such mixtures are characterized in that they comprise glycolide and a (predominant) lactide (e.g. L-lactide); and in that these components exhibit a phase equilibrium relationship which defines liquid-solid phase compartments represented by a projection of a phase diagram, where the lactide (e.g. L-lactide) and glycolide can crystallize respectively. The composition of the product stream is selected such that a substantially pure lactide stream (e.g. L-lactide stream) or substantially pure glycolide stream is formed by melt crystallization. The present process has the advantage of having limited number of processing steps, thereby reducing capital and energy costs, and providing for a simple and efficient process for the recovery of lactide or glycolide.

The present invention can be used for the separation of any mixtures comprising lactide(s) and glycolide. In other words, the mixtures of lactides and glycolide for use in a process of the invention may originate from any source, may for instance be obtained as a side-product, sidestream, or impurity from different production process, e.g. in processes for producing lactide, glycolide, PLA, PGA and PLGA.

In certain embodiments of a process of the invention, the mixture comprising lactide(s) and glycolide provided in step a) is obtained as a side-product in a process for preparing poly(lactic-co-glycolic acid) (PLGA) by ring-opening co-polymerization of lactide(s) and glycolide.

In certain other embodiments of a process of the invention, the mixture comprising lactide(s) and glycolide provided in step a) is obtained by thermal depolymerization of PLGA.

In certain other embodiments of a process of the invention, the mixture comprising lactide(s) and glycolide provided in step a) is prepared from a mixture comprising lactic acid and glycolic acid, and preferably is prepared by the steps of:
a1) providing a mixture comprising lactic acid and glycolic acid;
a2) subjecting said mixture comprising lactic acid and glycolic acid to a polycondensation under conditions effective to form lactic acid/glycolic acid co-oligomers; and,
a3) depolymerizing the lactic acid/glycolic acid co-oligomers formed in step a2) to form a mixture comprising lactide(s) and glycolide.

Lactic acid and glycolic acid and mixtures thereof may be obtained as described herein.

The independent and dependent claims set out particular and preferred features of the invention. Features from the dependent claims may be combined with features of the independent or other dependent claims as appropriate.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### DETAILED DESCRIPTION OF THE FIGURES

Figure 1 represents a phase diagram that was determined for L-lactide and glycolide in accordance with example 1.
Figure 2 represents a phase diagram that was determined for rac-lactide and glycolide in accordance with example 2.
Figure 3 represents a phase diagram that was determined for meso-lactide and glycolide in accordance with example 3.

### DETAILED DESCRIPTION

When describing the invention, the terms used are to be construed in accordance with the following definitions, unless a context dictates otherwise.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art.

The terms "comprising", "comprises" and "comprised of" as used herein are synonymous with "including", "includes" or "containing", "contains", and are inclusive or open-ended and do not exclude additional, non-recited members, elements, or method steps. It will be appreciated that the terms "comprising", "comprises" and "comprised of" as used herein comprise the terms "consisting of", "consists" and "consists of".

As used in the specification and the appended claims, the singular forms "a", "an," and "the" include plural referents unless the context clearly dictates otherwise. By way of example, "a step" means one step or more than one step.

Unless defined otherwise, all technical and scientific terms used herein have the same meaning as is commonly understood by one of skill in the art.

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of endpoints also includes the end point values themselves (e.g. from 1.0 to 5.0 includes both 1.0 and 5.0). Any numerical range recited herein is intended to include all sub-ranges subsumed therein.

The term "about" as used herein when referring to a measurable value such as a parameter, an amount, a temporal duration, and the like, is meant to encompass variations of +/-10% or less, preferably +/-5% or less, more preferably +/-1% or less, of and from the specified value, insofar such variations are appropriate to perform in the disclosed invention. It is to be understood that the value to which the modifier "about" refers is itself also specifically, and preferably, disclosed.

The terms "wt%", "vol%", or "mol%" refers to a weight percentage of a component, a volume percentage of a component, or molar percentage of a component, respectively, based on the total weight, the total volume of material, or total moles, that includes the component.

When describing the present invention, the terms used are to be construed in accordance with the following definitions, unless a context dictates otherwise.

Preferred statements (features) and embodiments and uses of this invention are set herein below. Each statement and embodiment of the invention so defined may be combined with any other statement and/or embodiment unless clearly indicated to the contrary. In particular, any feature indicated as being preferred or advantageous may be combined with any other feature or features or statements indicated as being preferred or advantageous. Hereto, the present invention is in particular captured by any one or any combination of one or more of the below numbered statements and embodiments, with any other aspect and/or embodiment.
1. Process for separating a mixture comprising lactide(s) and glycolide, wherein said process comprises the steps of:
   a) providing a mixture comprising lactide(s) and glycolide, wherein said mixture is provided in the molten state, and preferably wherein the total combined amount of lactide(s) and glycolide in said mixture is at least 70.0 wt%, based on the total weight of the mixture,
   b) determining the crystallization point of said mixture,
   c) determining a desired amount of lactide or of glycolide to be recovered from said mixture,
   d) selecting a suitable crystallization temperature (T_{c}) based on the desired amount of lactide or of glycolide to be recovered as determined in step c),
   e) subjecting said mixture to a melt crystallization at said temperature T_{c}, thereby obtaining a mother liquid and a crystallized product , wherein said crystallized product comprises crystalized lactide or crystallized glycolide, and
   f) recovering said crystallized product.
2. Process according to statement 1, wherein the total combined amount of lactide(s) and glycolide in said mixture is at least 70.0 wt%, based on the total weight of the mixture, such as at least 75.0 wt%, or at least 80.0 wt%, or at least 85.0 wt%, or at least 90.0 wt%, or at least 95.0 wt%, based on the total weight of the mixture.
3. Process according to any one of the preceding statements, wherein the lactide is selected from the group consisting of L-lactide, D-lactide, meso-lactide, and any mixtures thereof including rac-lactide.
4. Process according to any one of the preceding statements comprising the step of determining the predominant lactide in said mixture.
5. Process according to any one of the preceding statements, wherein the predominant lactide in said mixture is L-lactide, D-lactide, meso-lactide, or rac-lactide.
6. Process according to any one of the preceding statements, wherein the crystallization point of the mixture is determined by establishing a phase diagram of glycolide and the predominant lactide in said mixture.
7. Process according to any one of the preceding statements, wherein the phase diagram selected in step c) is a eutectic phase diagram established for the predominant lactide in said mixture and glycolide, and preferably is established by preparing a series of mixtures made of defined amounts of said predominant lactide and glycolide; and determining the crystallization points of said mixtures.
8. Process according any one of the preceding statements, wherein the crystallization temperature (T_{c}) is comprised in a range from 30 to 95 °C, or from 50 to 95°C, or from 50 to 80°C, or from 60 to 90°C.
9. Process according to any one of the preceding statements, comprising the step g) of further purifying the crystallized product recovered in step f) by melting said crystallized product to a molten product and supplying the molten product as a new mixture in step a), of the separation process of any one of the preceding statements, thereby recovering purified crystallized product and a new mother liquid from said recovered crystallized product.
10. Process according to any one of the preceding statements, wherein the process of statement 9 is repeated at most 10 times, preferably at most 5 times, more preferably at most 3 times.
11. Process according to any one of the preceding statements, comprising the step h) of supplying the mother liquid obtained in step e), as a new mixture in step a), of the separation process of any one of the preceding statements thereby recovering additional amounts of crystallized product and a new mother liquid.
12. Process according to any one of the preceding statements, wherein the process of statement 11 is repeated until a desired overall yield of crystallized product is achieved.
13. Process according to any one of the preceding statements, wherein at least 50%, or at least 60%, or at least 70%, or at least 75%, or at least 80%, or at least 85%, or at least 90% of the amount of lactide or of glycolide comprised in the mixture is recovered as crystallized product.
14. Process according to any one of the preceding statements, wherein melt crystallization comprises at least one layer crystallization, and preferably said layer crystallization comprises at least one falling film crystallization and/or at least one static crystallization.
15. Process according to any one of the preceding statements, wherein melt crystallization comprises at least one suspension crystallization.
16. Process according to any one of the preceding statements, wherein the crystallized product and optionally the purified crystallized product, are recovered by filtration, centrifugation, or another solid-liquid separation means, or any combinations thereof.
17. Process according to any one of the preceding statements 1 to 16, wherein lactide is separated from a mixture comprising lactide(s) and glycolide, and wherein said process comprises the steps of:
   a) providing a mixture comprising lactide(s) and glycolide, wherein said mixture is provided in the molten state,
   b) determining the crystallization point of said mixture,
   c) determining a desired amount of lactide be recovered from said mixture,
   d) selecting a suitable crystallization temperature (T_{c}) based on the desired amount of lactide to be recovered as determined in step c),
   e) subjecting said mixture to a melt crystallization at said temperature T_{c}, preferably a crystallization temperature (T_{c}) comprised between 30°C and 95°C, or between 50°C and 95°C, or between 60°C and 90°C, thereby obtaining a mother liquid and crystallized lactide ; and
   f) recovering said crystallized lactide.
18. Process any one of the preceding statements, wherein the total combined amount of lactide(s) and glycolide in said mixture is at least 70.0 wt%, based on the total weight of the mixture, such as at least 75.0 wt%, or at least 80.0 wt%, or at least 85.0 wt%, or at least 90.0 wt%, or at least 95.0 wt%, based on the total weight of the mixture.
19. Process according to any one of the preceding statements, wherein the lactide is selected from the group consisting of L-lactide, D-lactide, meso-lactide, and any mixtures thereof including rac-lactide.
20. Process according to any one of the preceding statements, wherein said crystallization temperature (T_{c}) is comprised between 30°C and 95°C, or between 50°C and 95°C, or between 60°C and 90°C.
21. Process according to any one of the preceding statements, comprising the step g) of further purifying the crystallized lactide recovered in step f) by melting said crystallized lactide to a molten product and supplying the molten product as a new mixture in step a), thereby recovering purified crystallized lactide and a new mother liquid from said recovered crystallized lactide
22. Process according to any one of the preceding statements, wherein the process of statement 21 is repeated at most 10 times, preferably at most 5 times, more preferably at most 3 times.
23. Process according to any one of the preceding statements, comprising the step h) of supplying mother liquids obtained in any one of the previous steps as a new mixture in step a), thereby recovering additional amounts (additional yield) of crystallized lactide and a new mother liquid.
24. Process according to any one of the preceding statements, wherein the process of statement 23 is repeated until a desired overall amount (overall yield) of the crystallized lactide is achieved.
25. Process according to any one of the preceding statements, wherein at least 50%, or at least 60%, or at least 70%, or at least 75%, or at least 80%, or at least 85%, or at least 90% of the amount of lactide comprised in the mixture is recovered as crystallized lactide.
26. Process according to any one of the preceding statements, wherein the recovered crystallized lactide comprises less than 1.0 wt%, preferably less than 0.5 wt%, of glycolide based on the total weight of the crystallized lactide.
27. Process according to any one of the preceding statements, wherein the recovered crystallized lactide comprises an amount (in wt%) of glycolide which is less than 0.5 times, such as less than 0.2 times, the amount (in wt%) of glycolide in the mixture.
28. The process according to any one of the preceding statements, wherein the purification factor for glycolide of the recovered crystallized lactide is in the range of 2 to 20.
29. Process according to any one of statements 1 to 16, wherein glycolide is separated from a mixture comprising lactide(s) and glycolide, wherein said process comprises the steps of:
   a) providing a mixture comprising lactide(s) and glycolide, wherein said mixture is provided in the molten state,
   b) determining the crystallization point of said mixture,
   c) determining a desired amount of glycolide to be recovered from said mixture,
   d) selecting a suitable crystallization temperature (T_{c}) based on the desired amount of glycolide to be recovered as determined in step c),
   e) subjecting said mixture to a melt crystallization at said temperature T_{c}, and preferably a crystallization temperature (T_{c}) comprised between 50°C and 80°C, such as between 60°C and 80°C, thereby obtaining a mother liquid and a crystallized glycolide comprising crystals of glycolide; and
   f) recovering said crystallized glycolide.
30. Process according to any one of statements 1 to 16 and 29, wherein the total combined amount of lactide(s) and glycolide in said mixture is at least 70.0 wt%, based on the total weight of the mixture, such as at least 75.0 wt%, or at least 80.0 wt%, or at least 85.0 wt%, or at least 90.0 wt%, or at least 95.0 wt%, based on the total weight of the mixture.
31. Process according to any one of statements 1 to 16 and 29 to 30, wherein the lactide is selected from the group consisting of L-lactide, D-lactide, meso-lactide, and any mixtures thereof including rac-lactide.
32. Process according to any one of statements 1 to 16 and 29 to 31, wherein said crystallization temperature (T_{c}) is comprised between 50°C and 80°C, such as between 60°C and 80°C.
33. Process according to any one of statements 1 to 16 and 29 to 32, comprising further purifying the crystallized glycolide recovered in step f) by melting said crystallized glycolide to a molten product and supplying the molten product as a new mixture in step a), thereby recovering purified crystallized glycolide and a new mother liquid from said recovered crystallized glycolide.
34. Process according to any one of statements 1 to 16 and 29 to 33, wherein the process of statement 33 is repeated at most 10 times, preferably at most 5 times, more preferably at most 3 times.
35. Process according to any one of statements 1 to 16 and 29 to 34, further comprising supplying mother liquid(s) obtained in any one of the previous steps, as a new mixture in step a), thereby recovering additional amounts of crystallized glycolide and a new mother liquid from said recovered crystallized glycolide.
36. Process according to any one of statements 1 to 16 and 29 to 35, wherein the process of statements 35 is repeated until a desired overall yield of the crystallized glycolide is achieved.
37. Process according to any one of statements 1 to 16 and 29 to 36, wherein at least 50%, or at least 60%, or at least 70%, or at least 75%, or at least 80%, or at least 85%, or at least 90% of the amount of glycolide comprised in said mixture is recovered as crystallized glycolide.
38. Process according to any one of statements 1 to 16 and 29 to 37, wherein the crystallized glycolide comprises less than 1.0 wt%, preferably less than 0.5 wt%, of lactide based on the total weight of the crystallized glycolide.
39. Process according to any one of statements 1 to 16 and 29 to 38, wherein the crystallized glycolide comprises an amount (in wt%) of lactide(s) which is less than 0.5 times, such as less than 0.2 times, the amount (in wt%) of lactide(s) in said mixture.
40. Process according to any one of statements 1 to 16 and 29 to 39, wherein the purification factor for lactide of the recovered crystallized glycolide is in the range of 2 to 20.
41. Process according to any one of the preceding statements, wherein the mixture comprising lactide(s) and glycolide provided in step a) is obtained as a side-product in a process for preparing poly(lactic-co-glycolic acid) (PLGA) by ring-opening co-polymerization of lactide(s) and glycolide.
42. Process according to any one of the preceding statements, wherein the mixture comprising lactide(s) and glycolide provided in step a) is recovered as a side-product in a process for the ring-opening co-polymerization of lactide(s) and glycolide into PLGA, by the steps of:
   i. subjecting lactide(s) and glycolide to a ring-opening co-polymerization under conditions effective to produce a reaction mixture comprising PLGA and unreacted lactide(s) and unreacted glycolide,
   ii. separating the unreacted lactide(s) and the unreacted glycolide from said PLGA, preferably by degassing the reaction mixture thereby recovering a vapor product comprising unreacted lactide(s) and unreacted glycolide; and
   iii. condensing said vapor product, thereby obtaining a mixture comprising lactide(s) and glycolide as a condensation product.
43. Process according to any one of the preceding statements, wherein the mixture comprising lactide(s) and glycolide provided in step a) is obtained by thermal depolymerization of PLGA.
44. Process according to any one of the preceding statements, wherein the mixture comprising lactide(s) and glycolide provided in step a) is prepared from a mixture comprising lactic acid and glycolic acid, and preferably is prepared by the steps of:
   a1) providing a mixture comprising lactic acid and glycolic acid;
   a2) subjecting said mixture comprising lactic acid and glycolic acid to a polycondensation under conditions effective to form lactic acid/glycolic acid co-oligomers; and,
   a3) depolymerizing the lactic acid/glycolic acid co-oligomers formed in step a2) to form a mixture comprising lactide(s) and glycolide.
45. Process according to statement 44, wherein the mixture comprising lactic acid and glycolic acid provided in step a1) is prepared by hydrolyzing PLA, PGA and PLGA in any suitable combination to yield lactic acid and glycolic acid.
46. Process according to statement 44 or 45, wherein the lactic acid provided in step a1) is prepared by fermentation.
47. Process according to any one of statements 44 to 46, wherein the glycolic acid provided in step a1) is prepared by fermentation.
48. Process according to any one of statements 44 to 47, wherein the glycolic acid provided in step a1) is prepared from synthesis gas.
49. Process according to any one of statements 44 to 48, wherein the mixture comprising lactic acid and glycolic acid provided in step a1) is prepared by
   i. subjecting lactide(s) and glycolide to a co-polymerization under conditions effective to produce a reaction mixture comprising PLGA and unreacted lactide(s) and unreacted glycolide,
   ii. separating the unreacted lactide(s) and the unreacted glycolide from said PLGA, preferably by degassing the reaction mixture thereby recovering a product stream comprising unreacted lactide(s) and unreacted glycolide; and
   iii. subjecting said product stream comprising unreacted lactide(s) and unreacted glycolide to hydrolysis under conditions effective to produce a mixture comprising lactic acid and glycolic acid.

The present invention is directed to a process for separating mixtures of lactide(s) and glycolide. More in particular, the present invention is directed to a process for recovering a lactide or glycolide from a mixture of lactide(s) and glycolide, by performing a melt crystallization at a suitable crystallization temperature, which is determined based on the composition of the mixture and a desired amount of lactide or of glycolide to be recovered from said mixture.

More in particular, the present invention relates to a process for separating a mixture comprising lactide(s) and glycolide, wherein said process comprises the steps of:
a) providing a mixture comprising lactide(s) and glycolide, wherein said mixture is provided in the molten state, and preferably wherein the total combined amount of lactide(s) and glycolide in said mixture is at least 70.0 wt%, based on the total weight of the mixture,
b) determining the crystallization point of said mixture,
c) determining a desired amount of lactide or of glycolide to be recovered from said mixture,
d) selecting a suitable crystallization temperature (T_{c}) based on the desired amount of lactide or of glycolide to be recovered as determined in step c),
e) subjecting said mixture to a melt crystallization at said temperature T_{c}, thereby obtaining a mother liquid and a crystallized product , wherein said crystallized product comprises crystalized lactide or crystallized glycolide,
f) recovering said crystallized product,
g) optionally further purifying the crystallized product recovered in step f) by melting said crystallized product to a molten product and supplying the molten product as a new mixture in step a) of the separation process of the invention, thereby recovering purified crystallized product and a new mother liquid from said recovered crystallized product, and
h) optionally supplying mother liquid(s) obtained in any one of the previous steps as a new mixture in step a), thereby recovering additional amounts of crystallized product and a new mother liquid.

The present invention provides a process for separating mixture comprising lactide(s) and glycolide. The mixture to be separated according to the present process is also referred herein as "initial" mixture, or "original" mixture. This is the mixture that is supplied at the start of the separation process.

The present process allows to obtain a crystallized product which comprises, or consists of, crystalized lactide or crystallized glycolide. The crystallized product obtained by the separation method may thus comprise or consist of crystallized lactide or crystallized glycolide. Whether crystallized lactide or crystallized glycolide is obtained may be depending on the composition of the initial mixture to be separated.

Crystallized lactide comprises, preferably essentially consists of "lactide crystals", and crystallized glycolide comprises, preferably essentially consists of "glycolide crystals". The present invention advantageously allows to obtain crystallized lactide or crystalized glycolide, respectively lactide crystals and glycolide crystals, of high purity, and at considerably yields.

As indicated above, a mixture to be separated in accordance with the present invention comprises glycolide and lactide(s).

Glycolide refers to the cyclic ester of two glycolic acid molecules.

Lactide refers to a cyclic ester of two lactic acid molecules. Lactide exists in three stereoisomeric forms, i.e., "L-lactide", which is a dimer of two L-lactic acid molecules, "D-lactide", which is a dimer of two D-lactic acid molecules and "meso-lactide", which is a dimer formed from one L-lactic acid molecule and one D-lactic acid molecule. The equal weight (50/50 wt%/wt%) mixture of L- and D-lactide is also known as "rac-lactide" or "racemic lactide".

The term "lactide" as used herein encompasses L-lactide, D-lactide, meso-lactide, or a mixture of two or more of the above, including rac-lactide (the 50/50 mixture between L- and D-lactide). In certain embodiments, lactides in a mixture to be separated according to the present process consist of only one type of lactide stereoisomer, e.g. L-lactide, or D-lactide, or meso-lactide. In certain other embodiments, lactides in a mixture to be separated according to the present process consist of two or more lactide stereoisomers, and for instance consist of a mixture of L-lactide and D-lactide, or a mixture of L-lactide and meso-lactide. In an example, lactides in a mixture to be separated according to the present separation process consist of rac-lactide.

In certain embodiments of the invention a mixture to be separated comprises a predominant lactide. The term "predominant" in reference to a lactide in a mixture refers in the present context to the stereoisomeric form of lactide that is present in greater mole percent than any other individual lactide stereoisomer available in the mixture. For instance the predominant lactide can be L-lactide, or D-lactide, or meso-lactide, or rac-lactide when equal amounts of Land D-lactide are present in the mixture. It will be understood that in embodiments in which the lactide in the mixture to be separated is rac-lactide, both L-lactide and D-lactide are present in the same mole percent. In such embodiments the rac-lactide (i.e. the 50/50 mixture of L-lactide and D-lactide) is to be understood as the "predominant lactide".

In certain embodiments of the present invention, it is preferred that the total combined amount of lactide(s) and glycolide in a mixture to be separated according to the invention at least 70.0 wt%, based on the total weight of the mixture, such as at least 75.0 wt%, or at least 80.0 wt%, or at least 85.0 wt%, or at least 90.0 wt%, or at least 95.0 wt%, or at least 99.0 wt%, or at least 99.5 wt%, based on the total weight of the mixture. Those skilled in the art will realize that mixtures to be separated may contain impurities, which may influence (decrease) crystallization points.

The mixture of lactide(s) and glycolide applied in step a) of the present process is in a molten state, i.e. in a liquid state.

Step b) of the present separation process involves the determination of the crystallization point of the mixture to be separated. The crystallization point of the mixture to be separated will depend on composition and concentration of the different components contained within the mixture, and e.g. depend on the type of lactide or lactides present in the mixture.

In certain preferred embodiments of the present process, the crystallization point of the mixture is determined by establishing a phase diagram of glycolide and the lactide which is the predominant lactide in said mixture.

Therefore the present process also involves a step of determining the predominant lactide in the mixture to be separated. This may be done using analytical methods known to the skilled man. In an example, the predominant lactide in a mixture of lactides and glycolide according to the invention is L-lactide. In another example, the predominant lactide in a mixture of lactides and glycolide according to the invention is D-lactide. In another example, the predominant lactide in a mixture of lactides and glycolide according to the invention is meso-lactide. In another example the predominant lactide in a mixture of lactides and glycolide to be separated according to the invention is rac-lactide.

A phase diagram in the context of the invention intends to refer to a type of chart used to show conditions at which thermodynamically distinct phases (such as solid, liquid, or gaseous states) occur and coexist at equilibrium and specifically, at which temperatures upon cooling a desired component crystallizes. A phase diagram for use in the present invention may refer to any suitable phase diagram such as a binary phase diagram (also referred to herein as eutectic phase diagram), a ternary phase diagram, a multicomponent phase diagram.

In certain preferred embodiments, a phase diagram selected in step c) of the present process is an eutectic phase diagram established for the predominant lactide (e.g. L-lactide) and glycolide. Such eutectic phase diagram is preferably established by preparing a series of mixtures made of defined amounts of said predominant lactide (e.g. L-lactide) and glycolide; and determining the crystallization points of said mixtures.

A next step in the present separation process involves the determination of a desired amount of lactide or of glycolide to be recovered from said mixture. Based on this desired amount of lactide or of glycolide to be recovered, a suitable crystallization temperature (T_{c}) can then be determined. In an example the desired amount of lactide or of glycolide to be recovered from said mixture and the corresponding crystallization temperature needed to arrive at this amount may be determined based on a phase diagram that was established for the mixture. The amount of crystals increases as the temperature is lowered, their amount can be predicted by the lever rule in binary equilibrium phase diagrams. The further away the original composition (initial mixture) is from the eutectic point, the more crystals may be harvested by cooling the mixture.

In according with the present separation process, it is preferred that the crystallization temperature (T_{c}) is comprised in a range from 30 to 95 °C. In certain embodiments of the present separation process, the crystallization temperature (T_{c}) is comprised in a range from, or from 50 to 95°C. In certain embodiments of the present separation process, the crystallization temperature (T_{c}) is comprised in a range from 50 to 80°C. In certain embodiments of the present separation process, the crystallization temperature (T_{c}) is comprised in a range from 60 to 90°C.

The mixture is then subjected to a melt crystallization step at the determined crystallization T_{c}, thereby such that a mother liquid and a crystallized product are obtained.

Crystallization involves purification of a chemical from a liquid mixture by solidification of the desired component. In melt crystallization, no solvent is added. The crystals are generated by cooling of the "melt". Melt crystallization can be performed in a variety of crystallization apparatuses.

In certain embodiments of the present process, melt crystallization may comprise at least one layer crystallization step. In preferred embodiments of the present process, the at least one layer crystallization step comprises at least one falling film crystallization step and/or at least one static crystallization step.

In certain embodiments of the present process, melt crystallization may comprise a falling film crystallization step. Falling film crystallization may be carried out in a falling film crystallizer. Such crystallizer typically consists of a system of vertical tubes. The collecting vessel below the tube sheet contains the liquid product (here mother liquid). The product and the heat transfer medium (HTM) both flow as a falling film. The mixture flows down on the inside surface of the tubes, whereas the liquid used for cooling and heating is distributed to wet the external surface of the tubes. During crystallization, cold heat transfer medium is used to chill the tubes and crystals adhere to the wall surface. These crystals may be further purified by so-called partial melting or sweating, which is induced by slightly raising the temperature of the heat transfer medium. Finally, the crystals are harvested by re-melting above the pure component melting point to provide a purified liquid. The product (mixture) and heat transfer medium distribution systems are designed to equalize flow through the tubes. Optimum performance is achieved through accurate control of the heating and cooling profiles and finetuning of the amount of partial melt.

In certain embodiments of the present process, melt crystallization may comprise at least one static crystallization step. Static crystallization may be carried out in a static crystallizer. Static crystallization is a process typically using vertical plates, heated, or cooled by an internal circulation of heat transfer medium. The plates are suspended in the liquid product (the mixture). Slow cooling of the heat transfer medium below a suitable crystallization temperature as determined herein of the stagnant product causes a layer of crystals to grow on the plates. Also in this type of layer crystallization, partial melt stages and product recovery stages are applied by increasing temperature.

Alternatively or additionally, melt crystallization applied in a process of the invention may also comprise at least one suspension crystallization step. Suspension crystallization is understood to be crystallization from a liquid due to cooling whilst applying agitation, wherein the crystals form a suspension with the liquid. Suspension crystallizations may be carried out in any type of stirred vessel, notable examples include a scraped-wall crystallizer, forced circulation crystallizer, and fluid bed crystallizer. The crystals in the suspension (or slurry) may be separated by any means, for example by filters, centrifuges, and hydraulic wash columns.

The crystallized product, i.e. comprising or consisting of crystals, formed during melt crystallization, may be separated from the mother liquid by any means known and appearing suitable to the skilled person. Preferably the crystallized product, and optionally the purified crystallized product (obtained as described herein), is recovered by means of filtration, centrifugation, or another solid-liquid separation means, or any combinations thereof. In an example the crystallized product may be separated and recovered by isolating the crystallized product (crystals), e.g. in a wash column, or a centrifugal filter, or a press filter.

In certain embodiments of the present process it is preferred that the crystallized product recovered in step f) of the process is further purified. To that end, certain embodiments of the present process involve a further step g) which comprises further purifying the crystallized product recovered in step f) by melting said crystallized product to a molten product and supplying the molten product as a new mixture in step a), thereby recovering purified crystallized product and a new mother liquid from said crystallized product.

The term "new mixture" as used herein intends to refer to a mixture that has a different composition as compared to the initial mixture as defined herein, i.e. the original mixture that is supplied at the start of the separation process.

The term "new mother liquid" as used herein used herein intends to a mother liquid that has a different composition as compared to the initial mother liquid, i.e. the mother liquid that is *directly* obtained from the initial mixture, as defined herein, after carrying out one first cycle the separation process of the invention.

The process of the invention, and in particular step g) of the process, may be repeated, such as at most 10 times, preferably at most 5 times, more preferably at most 3 times, and for instance at most 2 times or at most 1 times. The additional purification step(s) in accordance with step g) of the present separation process allow to improve the purity of the obtained crystallized product, if required. The term "purified crystallized product" used in this context intends to refer to a crystallized product (crystallized lactide or crystallized glycolide) that has higher purity (i.e. lower amounts of the respective non-desired dilactone) as compared to the crystallized product obtained in a previous cycle of the separation process.

For instance, when the crystallized product is crystallized lactide, it is preferred that the recovered crystallized lactide (i.e. the product that is recovered after carrying out the process of the invention) comprises less than 1.0 wt%, preferably less than 0.5 wt%, of glycolide based on the total weight of the crystallized lactide. Likewise, when the crystallized product is crystallized glycolide, it is preferred that the recovered crystallized glycolide (i.e. the product that is recovered after carrying out the process of the invention), comprises less than 1.0 wt%, preferably less than 0.5 wt%, of lactide based on the total weight of the crystallized glycolide.

In some embodiments, when the crystallized product is crystallized lactide, it is preferred that the crystallized lactide recovered with a process of the invention comprises an amount (in wt%) of glycolide which is less than 0.5 times, such as less than 0.4 times, or less than 0.3 times, or less than 0.2 times, the amount (in wt%) of glycolide in said (initial) mixture. In other words, it is preferred that the present separation process allows to obtain a purification factor for glycolide of the recovered crystallized lactide of at least 2, and for instance is a purification factor in the range of 2 to 20, such as from 3 to 17, or from 5 to 15.

Likewise, when the crystallized product is crystallized glycolide, it is preferred that the recovered crystallized glycolide comprises an amount (in wt%) of lactide(s) which is less than 0.5 times, such as less than 0.4 times, or less than 0.3 times, or less than 0.2 times, the amount (in wt%) of lactide(s) in the (initial) mixture. In other words, it is preferred that the present separation process allows to obtain a purification factor for lactide of the recovered crystallized glycolide of at least 2, and for instance is a purification factor in the range of 2 to 20, such as from 3 to 17, or from 5 to 15.

The term "purification factor" as used in this context may in general be defined as: 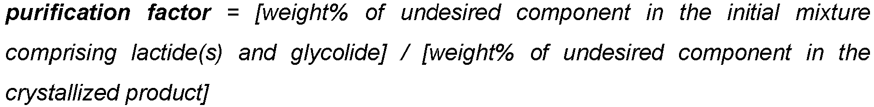 purification factor = [weight% of undesired component in the initial mixture comprising lactide(s) and glycolide] / [weight% of undesired component in the crystallized product]
wherein the "initial mixture" refers to the mixture as provided at the start of the separation process;
wherein the "crystallized product" is crystallized glycolide or crystallized lactide, and
wherein the "undesired component" refers to lactide or glycolide when the crystallized product is crystallized glycolide or crystallized lactide, respectively.

In certain preferred embodiments, the present process may also involve a step h) comprising supplying a mother liquid obtained in any one of the previous steps, as a new mixture in step a), thereby recovering additional amounts (additional yield) of crystallized product and a new mother liquid. The terms "new mixture" and "new mother liquid" are as defined hereinabove.

This additional step corresponds to a concentration of the mother liquid and allows to increase the amounts (yield) of recovered crystallized product. Preferably the process of the invention, and in particular step h) thereof is repeated until a desired overall yield of crystallized product is achieved. For instance, a desired overall yield of crystallized product may be an amount of the crystallized product (e.g. crystallized lactide or crystallized glycolide) that is at least 50%, as at least 60%, or at least 70%, or at least 75%, or at least 80%, or at least 85%, or at least 90% of the amount of the product (e.g. lactide or glycolide, respectively) that is comprised in said initial mixture.

In other words, in preferred embodiments of the present separation process at least 50%, such as at least 60%, or at least 70%, or at least 75%, or at least 80%, or at least 85%, or at least 90% of the amount of lactide or of glycolide comprised in the (initial) mixture is recovered as crystallized lactide or crystallized glycolide, respectively.

In certain embodiments, a process of the invention is provided wherein lactide is separated from a mixture comprising lactide(s) and glycolide, and wherein said process comprises the steps of:
a) providing a mixture comprising lactide(s) and glycolide, wherein said mixture is provided in the molten state, and preferably wherein the total combined amount of lactide(s) and glycolide in said mixture is at least 70.0 wt%, based on the total weight of the mixture, such as at least 75.0 wt%, or at least 80.0 wt%, or at least 85.0 wt%, or at least 90.0 wt%, or at least 95.0 wt%, based on the total weight of the mixture,
b) determining the crystallization point of said mixture,
c) determining a desired amount of lactide be recovered from said mixture,
d) selecting a suitable crystallization temperature (T_{c}) based on the desired amount of lactide to be recovered as determined in step c),
e) subjecting said mixture to a melt crystallization at said temperature T_{c}, preferably a crystallization temperature (T_{c}) comprised between 30°C and 95°C, or between 50°C and 95°C, or between 60°C and 90°C, thereby obtaining a mother liquid and crystallized lactide; and
f) recovering said crystallized lactide.

In an example, the predominant lactide in a mixture of lactides and glycolide according to the invention is L-lactide. In another example, the predominant lactide in a mixture of lactides and glycolide according to the invention is D-lactide. In another example, the predominant lactide in a mixture of lactides and glycolide according to the invention is meso-lactide.

In preferred embodiments, the present process further comprises the step g) of further purifying the crystallized lactide recovered in step f) by melting said crystallized lactide to a molten product and supplying the molten product as a new mixture in step a), thereby recovering purified crystallized lactide and a new mother liquid from said crystallized lactide. This step g) may be repeated, such as at most 10 times, preferably at most 5 times, more preferably at most 3 times, and for instance at most 2 times or at most 1 times. These additional purification step(s) in accordance with step g) of the present separation method for separating lactide allow to improve the purity of the obtained crystallized lactide, if required. For instance, it is preferred that the recovered crystallized lactide (i.e. the product that is recovered after carrying out the process of the invention) comprises less than 1.0 wt%, preferably less than 0.5 wt%, of glycolide based on the total weight of the crystallized lactide.

In some embodiments, it is preferred that the crystallized lactide recovered with a process of the invention comprises an amount (in wt%) of glycolide which is less than 0.5 times, such as less than 0.4 times, or less than 0.3 times, or less than 0.2 times, the amount (in wt%) of glycolide in said (initial) mixture. In other words, it is preferred that the present separation process allows to obtain a purification factor for glycolide of the recovered crystallized lactide of at least 2, and for instance is a purification factor in the range of 2 to 20, such as from 3 to 17, or from 5 to 15.

Additionally or alternatively, preferred embodiments of the present process further comprises the step h) of supplying mother liquid(s) obtained in any one of the previous steps of the process, as a new mixture in step a), thereby recovering additional amounts of crystallized lactide and a new (concentrated) mother liquid. The additional concentration step of mother liquid(s) in accordance with step h) of the present separation method allows to increase the amount of recovered crystallized lactide. Optionally, step h) may be repeated until a desired overall yield of crystallized lactide is achieved. Preferably, a desired overall yield of crystallized lactide may be an amount that is at least 50%, as at least 60%, or at least 70%, or at least 75%, or at least 80%, or at least 85%, or at least 90% of the amount of the lactide that is comprised in the initial mixture. In other words, in preferred embodiments of the present lactide separation process at least 50%, such as at least 60%, or at least 70%, or at least 75%, or at least 80%, or at least 85%, or at least 90% of the amount of lactide comprised in the (initial) mixture is recovered as crystallized lactide.

In certain preferred embodiments of the present process, a separation process is provided wherein L-lactide is separated from a mixture comprising L-lactide and optionally other lactide(s), and glycolide, and wherein said process comprises the steps of:
a) providing a mixture comprising L-lactide and optionally other lactide(s) and glycolide, wherein said mixture is provided in the molten state, and
   preferably wherein the total combined amount of L-lactide and glycolide in said mixture is at least 70.0 wt%, based on the total weight of the mixture, such as at least 75.0 wt%, or at least 80.0 wt%, or at least 85.0 wt%, or at least 90.0 wt%, or at least 95.0 wt%, based on the total weight of the mixture,
b) determining the crystallization point of said mixture,
c) determining a desired amount of L-lactide to be recovered from said mixture,
d) selecting a suitable crystallization temperature (T_{c}) based on the desired amount of L-lactide to be recovered as determined in step c),
e) subjecting said mixture to a melt crystallization at said temperature T_{c}, preferably a crystallization temperature (T_{c}) comprised between 30°C and 95°C, or between 50°C and 95°C, or between 60°C and 90°C, thereby obtaining a mother liquid and crystallized L-lactide comprising crystals of L-lactide; and
f) recovering said crystallized L-lactide,
g) optionally further purifying the crystallized L-lactide recovered in step f) by melting said crystallized L-lactide to a molten L-lactide and supplying the molten L-lactide as a new mixture in step a), thereby recovering purified crystallized L-lactide and a new mother liquid from said crystallized L-lactide, and
h) optionally supplying mother liquid(s) obtained in any one of the previous steps, as a new mixture in step a), thereby recovering additional amounts of crystallized L-lactide and a new mother liquid.

In preferred embodiments of the process, the recovered crystallized L-lactide comprises an amount (in wt%) of glycolide which is less than 0.5 times, such as less than 0.2 times, the amount (in wt%) of glycolide in the mixture. In preferred embodiments of the process, the purification factor for glycolide of the recovered crystallized L-lactide is in the range of 2 to 20, such as from 3 to 17, or from 5 to 15.

In certain other embodiments, a process of the invention is provided wherein glycolide is separated from a mixture comprising lactide(s) and glycolide, and wherein said process comprises the steps of:
a) providing a mixture comprising lactide(s) and glycolide, wherein said mixture is provided in the molten state,
   preferably wherein the total combined amount of lactide(s) and glycolide in said mixture is at least 70.0 wt%, based on the total weight of the mixture, such as at least 75.0 wt%, or at least 80.0 wt%, or at least 85.0 wt%, or at least 90.0 wt%, or at least 95.0 wt%, based on the total weight of the mixture,
b) determining the crystallization point of said mixture,
c) determining a desired amount of glycolide to be recovered from said mixture,
d) selecting a suitable crystallization temperature (T_{c}) based on the desired amount of glycolide to be recovered as determined in step c),
e) subjecting said mixture to a melt crystallization at said temperature T_{c}, and preferably a crystallization temperature (T_{c}) comprised between 50°C and 80°C, such as between 60°C and 80°C, thereby obtaining a mother liquid and a crystallized glycolide comprising crystals of glycolide; and
f) recovering said crystallized glycolide,
g) optionally further purifying the crystallized glycolide recovered in step f) by melting said crystallized glycolide to a molten product and supplying the molten product as a new mixture in step a), thereby recovering purified crystallized glycolide and a new mother liquid from said crystallized glycolide, and
h) optionally supplying mother liquids obtained in any one of the previous steps, as a new mixture in step a), thereby recovering additional amounts of crystallized glycolide and a new mother liquid.

In preferred embodiments, the present process further comprises the step g) of further purifying the crystallized glycolide recovered in step f) by melting said crystallized glycolide to a molten product and supplying the molten product as a new mixture in step a), thereby recovering purified crystallized glycolide and a new mother liquid from the recovered crystallized glycolide. This step g) may be repeated, such as at most 10 times, preferably at most 5 times, more preferably at most 3 times, such as at most 2 times or at most 1 times. These additional purification step(s) in accordance with step g) of the present separation method for separating glycolide allow to improve the purity of the obtained crystallized lactide, if required. For instance, it is preferred that the recovered crystallized glycolide (i.e. the product that is recovered after carrying out the process of the invention) comprises less than 1.0 wt%, preferably less than 0.5 wt%, of lactide(s) based on the total weight of the crystallized glycolide.

In some embodiments, it is preferred that the crystallized glycolide recovered with a process of the invention comprises an amount (in wt%) of lactide(s) which is less than 0.5 times, such as less than 0.4 times, or less than 0.3 times, or less than 0.2 times, the amount (in wt%) of lactide(s) in said (initial) mixture. In other words, it is preferred that the present separation process allows to obtain a purification factor for lactide of the recovered crystallized glycolide of at least 2, and for instance is a purification factor in the range of 2 to 20, such as from 3 to 17, or from 5 to 15.

Additionally or alternatively, preferred embodiments of the present process further comprises the step h) of supplying mother liquids obtained in any one of the previous steps, as a new mixture in step a), thereby recovering additional amounts (additional yield) of crystallized glycolide and a new, further concentrated, mother liquid. The additional concentration steps of mother liquid(s) in accordance with step h) of the present glycolide separation method allows to increase the amounts (yield) of recovered crystallized glycolide. Optionally the process of the invention, and in particular step h) thereof is repeated until a desired overall yield of crystallized glycolide is achieved. Preferably, a desired overall yield of crystallized glycolide may be an amount that is at least 50%, as at least 60%, or at least 70%, or at least 75%, or at least 80%, or at least 85%, or at least 90% of the amount of the glycolide that is comprised in the initial mixture. In other words, in preferred embodiments of the present separation process for separating glycolide at least 50%, such as at least 60%, or at least 70%, or at least 75%, or at least 80%, or at least 85%, or at least 90% of the amount of glycolide comprised in the mixture is recovered as crystallized glycolide.

The present process involves a first step in which mixtures of lactide(s) and glycolide are provided. Mixtures comprising lactide(s) and glycolide for separation in accordance with a process as defined herein may be obtained from various sources and/or as side-products or side streams from various processes, e.g. for making PLA, PGA or PLGA.

The term polylactide (PLA) refers to a polymer of lactic acid units, e.g. produced by ring-opening polymerization of L-lactide, D-lactide, meso-lactide or a mixture of two or three of these lactides. It can also be a mixture of polymers from the named pure or mixed lactides. PLA as a rule has a number average molecular weight >10,000 g/mol.

The term polyglycolide (PGA) refers to a polymer of glycolic acid units, e.g. produced by ring-opening polymerization of the glycolide. As a rule, PGA has a number average molecular weight >10,000 g/mol.

The term poly(lactic-co-glycolic acid (PLGA) refers to a copolymer of glycolic acid and lactic acid. PLGA polymers can be random or block copolymers.

In one example, a mixture of lactide(s) and glycolide as provided in step a) in the present process may be obtained starting from PLGA.

In certain embodiments, a mixture of lactide(s) and glycolide as provided in step a) in the present process may be obtained by a thermal depolymerization of PLGA. For instance, PLGA may be subjected to catalytic backbiting by adding excess catalyst and/or by applying temperatures above 200°C during its production

In certain other examples, mixtures comprising lactide(s) and glycolide may be obtained as side products or side streams which are formed during the production of PLGA. For instance, in some embodiments, a mixture of lactide(s) and glycolide as provided in step a) in the present process may be prepared or recovered as a side-product in a process for the ring-opening co-polymerization of lactide(s) and glycolide into PLGA. Preferably, in such example, a mixture of lactide(s) and glycolide as provided in step a) in the present separation process is preparing by the steps of:
I. subjecting lactide(s) and glycolide to a ring-opening co-polymerization under conditions effective to produce a reaction mixture comprising PLGA and unreacted lactide(s) and unreacted glycolide,
II. separating the unreacted lactide(s) and the unreacted glycolide from said PLGA, preferably by degassing the reaction mixture thereby recovering a vapor product comprising unreacted lactide(s) and unreacted glycolide; and
III. condensing said vapor product, thereby obtaining a mixture comprising lactide(s) and glycolide as a condensation product.

Those skilled in the art will recognize that when producing PLGA copolymers with conventional catalysts like group IV alkoxides, tin oxides and tin alkanoates, specifically tin octoate, typically glycolide ring-opening polymerization proceeds at faster rates than lactide under the same conditions. PLGA co-polymerizations tend to show a gradient-like co-polymerization behavior and the reaction product before degassing can typically contain very small amounts of residual glycolide (<0.5 wt%) whereas lactide, dependent on the final polymerization temperature, can constitute about 2-5% of the mass. As such in practice such gradient co-polymerizations will allow recovery of a condensed lactide stream with only minor amounts of glycolide. If so desired, the co-polymerization may be altered by multiple injections of glycolide and the amount of residual glycolide in the product sent to the degassing stages of the process may increase. Also, catalysts may be employed to change the copolymerization kinetics.

In certain embodiments, mixtures comprising lactide(s) and glycolide may be obtained starting from mixtures of lactic acid and glycolic acid.

For instance a mixture of lactide(s) and glycolide as provided in step a) is prepared from a mixture of lactic acid and glycolic acid by the steps of:
a1) providing a mixture of lactic acid and glycolic acid;
a2) subjecting said mixture of lactic acid and glycolic acid to a polycondensation under conditions effective to form lactic acid/glycolic acid co-oligomers; and,
a3) depolymerizing the lactic acid/glycolic acid co-oligomers formed in step a2) to form a mixture comprising lactide(s) and glycolide.

Mixtures of lactic acid and the glycolic acid as applied in step a1) may be obtained in various ways.

In an example, mixtures of glycolic acid and lactic acid can be prepared by hydrolyzing PLA, PGA, and PLGA in any suitable combination to yield glycolic acid and lactic acid. For instance, a mixture of glycolic acid and lactic acid can be prepared by hydrolyzing PLA and PGA. For instance, a mixture of glycolic acid and lactic acid can be prepared by hydrolyzing PLA and PLGA. For instance, a mixture of glycolic acid and lactic acid can be prepared by hydrolyzing PGA and PLGA. For instance, a mixture of glycolic acid and lactic acid can be prepared by hydrolyzing PLGA. For instance, a mixture of glycolic acid and lactic acid can be prepared by hydrolyzing PLA, PGA, and PLGA.

In another example lactic acid and/or glycolic acid may be prepared by fermentation.

In yet another example glycolic acid may also be prepared starting from synthesis gas (syngas) and oxygen, for instance by via the preparation of a dimethyl oxalate intermediate.

In certain embodiments, mixtures of lactic acid and the glycolic acid as applied in step a1) may be prepared by a process comprising the steps of:
(i) subjecting lactide(s) and glycolide to a co-polymerization under conditions effective to produce a reaction mixture comprising PLGA and unreacted lactide(s) and unreacted glycolide,
(ii) separating the unreacted lactide(s) and the unreacted glycolide from said PLGA, preferably by degassing the reaction mixture thereby recovering a product stream comprising unreacted lactide(s) and unreacted glycolide; and
(iii) subjecting said product stream comprising unreacted lactide(s) and unreacted glycolide to hydrolysis under conditions effective to produce a mixture comprising lactic acid and glycolic acid.

Step a2) of the above method involves subjecting said mixture of lactic acid and glycolic acid to a polycondensation under conditions effective to form lactic acid/glycolic acid co-oligomers. In an example, polycondensation conditions applied in step a2) may include heating to temperatures of about 100 to 180°C, and applying a pressure of about 10 to 500 mbar. Preferably no catalyst is applied during such polycondensation. A skilled person is familiar with conditions that are suitable for arriving at an effective polycondensation reaction.

Step a3) of the above method involves a depolymerization of the lactic acid/glycolic acid co-oligomers formed in step a2) to form a mixture comprising lactide(s) and glycolide. In an example, depolymerization conditions applied in step a3) may include heating to a temperatures of about 175 to 225°C, and applying a pressure of about 2- 50 mbar, in the presence of a suitable catalyst. A skilled person is familiar with conditions and catalysts that can be applied for obtaining effective depolymerization. It will also be understood that mixtures resulting from the above process, including the depolymerization step a3), may comprise significant amounts of compounds different from lactide and glycolide, e.g. mixed dilactones such as e.g. 3-methyl glycolide, and the like. Also this type of mixtures, i.e. comprising lactides, glycolide and other components such as other dilactones, may be separated by applying a separation process according to the invention.

The following examples serve to merely illustrate the invention and should not be construed as limiting the scope in any way. While the invention has been shown in only some of its forms, it should be apparent to those skilled in the art that it is not so limited, but is susceptible to various changes and modifications without departing from the scope of the invention.

### EXAMPLES

### EXAMPLE 1: L-lactide and glycolide

In this example, a phase diagram was established for L-lactide and glycolide.

A mixture of glycolide and L-lactide was prepared. The two monomers were weighed at the right mass percentages in PE sealing bags on a 200 gram scale. After weighing they were doubly sealed into an aluminum bag. The samples were placed in a freezer and allowed to heat to room temperature before use.

Crystallization experiments were performed in a 500 mLjacketed vessel equipped with stirring rod and water heating bath which was programmable. Into the vessel, the full 200 grams of monomer mixture was placed and allowed to heat in about 20 minutes to 98°C. Cooling was then done in steps of 5K every 5 minutes, while constantly adding seeding crystals (to avoid undercooling). The seeding crystals generally persisted when the temperature was within 10K of the later determined crystallization point. After a first rough indication of the crystallization point was determined, the mixture was again heated to 98°C and subsequently cooled to 3-5 degrees above the earlier determined temperature. A final cooling ramp of 2K/h was then used to find the accurate crystallization temperature (using again persisting seeding crystals).

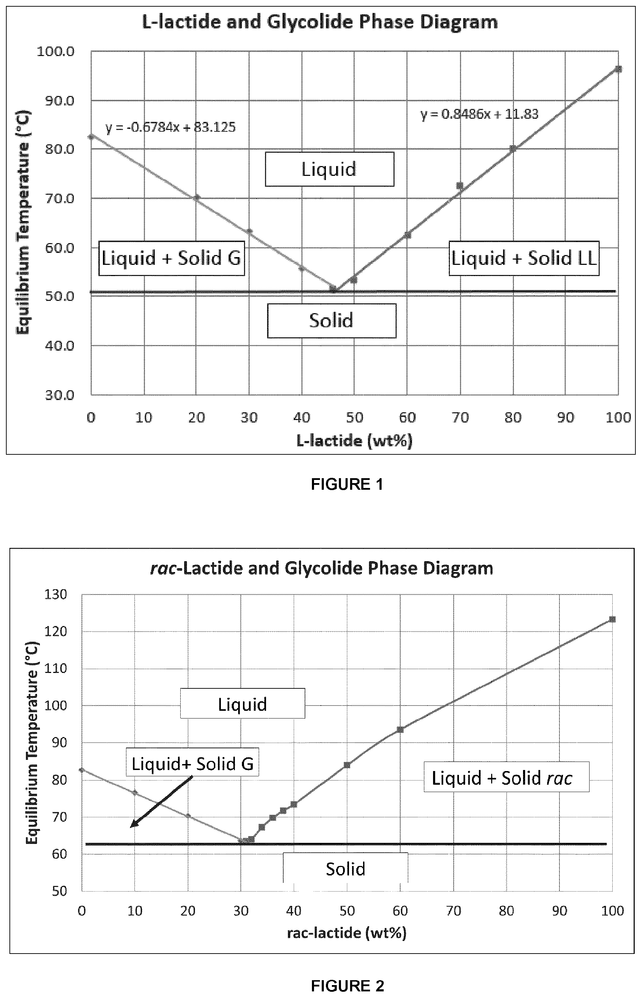

The crystallization points were determined and plotted in the phase diagram shown in **Figure 1****.** The eutectic point was determined to be located at about 46 wt% lactide and about 51°C.

### EXAMPLE 2: rac-lactide and glycolide

In this example, a phase diagram for rac-lactide and glycolide was established. A mixture of glycolide and rac-lactide was prepared in a same way as described in example 1. Crystallization experiments were performed in a same way as reported for example 1. The crystallization points were determined and plotted in the phase diagram shown in **Figure 2****.** The eutectic point was determined to be located at about 31 wt% rac-lactide and about 61°C.

### EXAMPLE 3: meso-lactide and glycolide

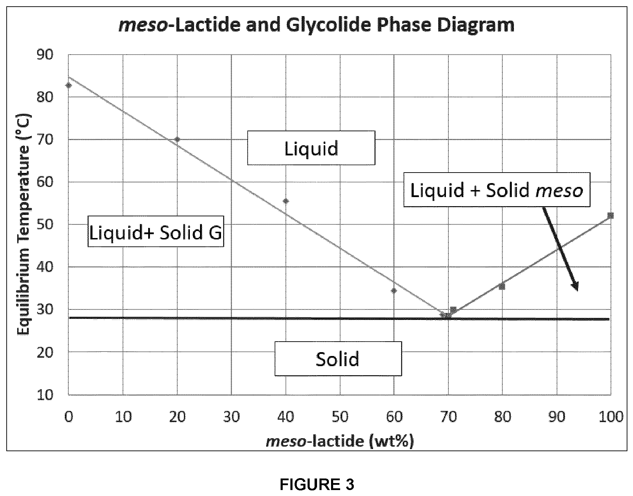

In this example, a phase diagram was established for meso-lactide and glycolide. A mixture of glycolide and meso-lactide was prepared in a same was as described in example 1. Crystallization experiments were performed in a same way as reported for example 1. The crystallization points were determined and plotted in the phase diagram shown in **Figure 3****.** The eutectic point was determined to be located at about 70wt% meso-lactide and about 29°C.

### EXAMPLE 4: multiple cycles of suspension crystallization

In this example a 250 ml jacketed vessel equipped with thermostat and a magnetic stirrer was used to perform melt crystallizations between L-lactide and glycolide.

A 90wt%/10wt% mixture of L-lactide/glycolide was made by adding 180 gram L-lactide (Lumilact L, TotalEnergies Corbion) and 20 gram glycolide (glycolide, PJ Chem) to the jacketed vessel. The mixture is molten at 98°C under 300 rpm stirring. When molten the mixture was cooled at 20°C/h to 2°C above the theoretical crystallization temperature. The cooling rate was then changed to 2°C/h and after every 1°C lactide seeds were added. At 89°C, the lactide seeds persisted and the mixture was maintained for 30 minutes. After 30 minutes, cooling was continued at 2°C/h to 78°C (based on the phase diagram in Figure 1 this was the expected temperature at which about 50 wt% of crystals are formed, with wt% based on the total original amount of material). Crystals and mother liquor (mother liquid) were separated by means of a basket centrifuge (Hermle Sieva, 6000rpm) with a 25 micron filter. The crystals were collected and sealed in a PE bag for storage.

The next day, the obtained crystals were subjected to a second crystallization performed in the same way, final crystallization temperature being 92°C. Samples were methylated and the content of methyl lactate and methyl glycolate were determined using gas chromatography. Using a pure glycolide calibration curve, glycolide contents were then determined and results are reported in Table 1.

**Table 1: Glycolide contents of subsequent suspension crystallizations**

| **Sample description** | **Glycolide content (GC, wt%)** |
|---|---|
| crystals after first crystallization | 0.7 |
| mother liquor after first crystallization | 17 |
| crystals after second crystallization | 0.1 |
| mother liquor after second crystallization | 1.2 |

## Claims

1. Process for separating a mixture comprising lactide(s) and glycolide, wherein said process comprises the steps of:
a) providing a mixture comprising lactide(s) and glycolide, wherein said mixture is provided in the molten state, and preferably wherein the total combined amount of lactide(s) and glycolide in said mixture is at least 70.0 wt%, based on the total weight of the mixture,
b) determining the crystallization point of said mixture,
c) determining a desired amount of lactide or of glycolide to be recovered from said mixture,
d) selecting a suitable crystallization temperature (T_{c}) based on the desired amount of lactide or of glycolide to be recovered as determined in step c),
e) subjecting said mixture to a melt crystallization at said temperature T_{c}, thereby obtaining a mother liquid and a crystallized product , wherein said crystallized product comprises crystalized lactide or crystallized glycolide, and
f) recovering said crystallized product.

2. Process according to claim 1, wherein the crystallization point of the mixture is determined by establishing a phase diagram of glycolide and the lactide which is the predominant lactide in said mixture.

3. Process according any one of the preceding claims, wherein the crystallization temperature (T_{c}) is comprised in a range from 30 to 95 °C, or from 50 to 95°C, or from 50 to 80°C, or from 60 to 90°C.

4. Process according to any one of the preceding claims, wherein the crystallized product recovered in step f) is further purified by melting said crystallized product to a molten product and supplying the molten product as a new mixture in step a) of the separation process of any one of the preceding claims, thereby recovering purified crystallized product and a new mother liquid from said recovered crystallized product.

5. Process according to any one of the preceding claims, wherein the mother liquid obtained in step e), is supplied as a new mixture in step a) of the separation process of any one of the preceding claims, thereby recovering additional amounts of crystallized product and a new mother liquid.

6. Process according to any one of the preceding claims, wherein at least 50%, or at least 60%, or at least 70%, or at least 75%, or at least 80%, or at least 85%, or at least 90% of the amount of lactide or of glycolide comprised in the mixture is recovered as crystallized product.

7. Process according to any one of the preceding claims, wherein melt crystallization comprises
- at least one layer crystallization, and preferably said layer crystallization comprises at least one falling film crystallization and/or at least one static crystallization, and/or
- at least one suspension crystallization.

8. Process according to any one of the preceding claims, wherein the crystallized product, and optionally the purified crystallized product, are recovered by filtration, centrifugation, or another solid-liquid separation means, or any combinations thereof.

9. Process according to any one of the preceding claims, wherein lactide is separated from a mixture comprising lactide(s) and glycolide, and wherein said process comprises the steps of:
a) providing a mixture comprising lactide(s) and glycolide, wherein said mixture is provided in the molten state, and preferably wherein the total combined amount of lactide(s) and glycolide in said mixture is at least 70.0 wt%, based on the total weight of the mixture,
b) determining the crystallization point of said mixture,
c) determining a desired amount of lactide to be recovered from said mixture,
d) selecting a suitable crystallization temperature (T_{c}) based on the desired amount of lactide to be recovered as determined in step c),
e) subjecting said mixture to a melt crystallization at said temperature T_{c}, preferably a crystallization temperature (T_{c}) comprised between 30°C and 95°C, or between 50°C and 95°C, or between 60°C and 90°C, thereby obtaining a mother liquid and crystallized lactide; and
f) recovering said crystallized lactide,
g) optionally further purifying the crystallized lactide recovered in step f) by melting said crystallized lactide to a molten product and supplying the molten product as a new mixture in step a), thereby recovering purified crystallized lactide and a new mother liquid from said recovered crystallized lactide, and
h) optionally supplying mother liquid(s) obtained in any one of the previous steps, as a new mixture in step a), thereby recovering additional amounts of crystallized lactide and a new mother liquid.

10. Process according to claim 9, wherein the crystallized lactide comprises an amount (in wt%) of glycolide which is less than 0.5 times, such as less than 0.2 times, the amount (in wt%) of glycolide in the mixture.

11. The process according to claim 9 or 10, wherein the purification factor for glycolide of the crystallized lactide is in the range of 2 to 20.

12. Process according to any one of claims 1 to 8, wherein glycolide is separated from a mixture comprising lactide(s) and glycolide, wherein said process comprises the steps of:
a) providing a mixture comprising lactide(s) and glycolide, wherein said mixture is provided in the molten state, and preferably wherein the total combined amount of lactide(s) and glycolide in said mixture is at least 70.0 wt%, based on the total weight of the mixture,
b) determining the crystallization point of said mixture,
c) determining a desired amount of glycolide to be recovered from said mixture,
d) selecting a suitable crystallization temperature (T_{c}) based on the desired amount of glycolide to be recovered as determined in step c),
e) subjecting said mixture to a melt crystallization at said temperature T_{c}, and preferably a crystallization temperature (T_{c}) comprised between 50°C and 80°C, such as between 60°C and 80°C, thereby obtaining a mother liquid and a crystallized glycolide comprising crystals of glycolide; and
f) recovering said crystallized glycolide,
g) optionally further purifying the crystallized glycolide recovered in step f) by melting said crystallized glycolide to a molten product and supplying the molten product as a new mixture in step a), thereby recovering purified crystallized glycolide and a new mother liquid from said recovered crystallized glycolide, and
h) optionally supplying mother liquid(s) obtained in any one of the previous steps, as a new mixture in step a), thereby recovering additional amounts of crystallized glycolide and a new mother liquid.

13. Process according to any one of claims 1 to 8 and 12, wherein the crystallized glycolide comprises an amount (in wt%) of lactide(s) which is less than 0.5 times, such as less than 0.2 times, the amount (in wt%) of lactide(s) in the mixture.

14. The process according to any one of claims 1 to 8 and claims 12 to 13, wherein the purification factor for lactide of the crystallized glycolide is in the range of 2 to 20.

15. Process according to any one of the preceding claims, wherein the mixture comprising lactide(s) and glycolide provided in step a) is obtained as a side-product in a process for preparing poly(lactic-co-glycolic acid) (PLGA) by ring-opening co-polymerization of lactide(s) and glycolide.

16. Process according to any one of the preceding claims, wherein the mixture comprising lactide(s) and glycolide provided in step a) is obtained by thermal depolymerization of PLGA.

17. Process according to any one of the preceding claims, wherein the mixture comprising lactide(s) and glycolide provided in step a) is prepared from a mixture comprising lactic acid and glycolic acid, and preferably is prepared by the steps of:
a1) providing a mixture comprising lactic acid and glycolic acid;
a2) subjecting said mixture comprising lactic acid and glycolic acid to a polycondensation under conditions effective to form lactic acid/glycolic acid co-oligomers; and,
a3) depolymerizing the lactic acid/glycolic acid co-oligomers formed in step a2) to form a mixture comprising lactide(s) and glycolide.

## Patentansprüche

1. Verfahren zum Trennen einer Mischung, die Lactid(e) und Glycolid aufweist, wobei das besagte Verfahren die Schritte aufweist:
a) Bereitstellen einer Mischung, die Lactid(e) und Glycolid aufweist, wobei die besagte Mischung im geschmolzenen Zustand bereitgestellt wird und vorzugsweise wobei die kombinierte Gesamtmenge an Lactid(en) und Glycolid in besagter Mischung mindestens 70,0 Gew.-%, bezogen auf das Gesamtgewicht der Mischung, ist,
b) Ermitteln des Kristallisationspunkts der besagten Mischung,
c) Ermitteln einer gewünschten Menge an Lactid oder an Glycolid, die aus der besagten Mischung zurückzugewinnen ist,
d) Auswählen einer geeigneten Kristallisationstemperatur (T_{c}) basierend auf der gewünschten Menge an Lactid oder Glycolid, die zurückzugewinnen ist, wie in Schritt c) ermittelt,
e) Unterziehen der besagten Mischung einer Schmelzkristallisation bei der besagten Temperatur T_{c}, wodurch eine Mutterflüssigkeit und ein kristallisiertes Produkt erlangt werden, wobei das besagte kristallisierte Produkt kristallisiertes Lactid oder kristallisiertes Glycolid aufweist, und
f) Zurückgewinnen des besagten kristallisierten Produkts.

2. Verfahren gemäß Anspruch 1, wobei der Kristallisationspunkt der Mischung ermittelt wird mittels Erstellens eines Phasendiagramms von Glykolid und dem Lactid, das das vorherrschende Lactid in der Mischung ist.

3. Verfahren gemäß irgendeinem der vorstehenden Ansprüche, wobei die Kristallisationstemperatur (T_{c}) in einem Bereich von 30 bis 95 °C oder von 50 bis 95 °C oder von 50 bis 80 °C oder von 60 bis 90 °C ist.

4. Verfahren gemäß irgendeinem der vorstehenden Ansprüche, wobei das in Schritt f) zurückgewonnene kristallisierte Produkt weiter aufgereinigt wird mittels Schmelzens des besagten kristallisierten Produkts zu einem geschmolzenen Produkt und Zuführen des geschmolzenen Produkts als neue Mischung in Schritt a) des Trennverfahrens gemäß irgendeinem der vorstehenden Ansprüche, wodurch aufgereinigtes kristallisiertes Produkt und eine neue Mutterflüssigkeit aus dem zurückgewonnenen kristallisierten Produkt zurückgewonnen werden.

5. Verfahren gemäß irgendeinem der vorstehenden Ansprüche, wobei die in Schritt e) erlangte Mutterflüssigkeit als eine neue Mischung in Schritt a) des Trennverfahrens gemäß irgendeinem der vorstehenden Ansprüche zugeführt wird, wodurch zusätzliche Mengen an kristallisiertem Produkt und eine neue Mutterflüssigkeit zurückgewonnen werden.

6. Verfahren gemäß irgendeinem der vorstehenden Ansprüche, wobei mindestens 50% oder mindestens 60%, oder mindestens 70%, oder mindestens 75%, oder mindestens 80%, oder mindestens 85%, oder mindestens 90% der in der Mischung erlangten Menge an Lactid oder Glycolid als kristallisiertes Produkt zurückgewonnen werden.

7. Verfahren gemäß irgendeinem der vorstehenden Ansprüche, wobei die Schmelzkristallisation aufweist:
- mindestens eine Schichtkristallisation und wobei die besagte Schichtkristallisation vorzugsweise mindestens eine Fallfilmkristallisation und/oder mindestens eine statische Kristallisation aufweist, und/oder
- mindestens eine Suspensionskristallisation.

8. Verfahren gemäß irgendeinem der vorstehenden Ansprüche, wobei das kristallisierte Produkt, und optional das aufgereinigte kristallisierte Produkt, mittels Filtration, Zentrifugation, oder eines anderen Mittels zur Fest-Flüssig-Trennung oder irgendwelcher Kombinationen davon zurückgewonnen werden.

9. Verfahren gemäß irgendeinem der vorstehenden Ansprüche, wobei Lactid aus einer Mischung, die Lactid(e) und Glycolid aufweist, abgetrennt wird, und wobei das Verfahren die Schritte aufweist:
a) Bereitstellen einer Mischung, die Lactid(e) und Glycolid aufweist, wobei die besagte Mischung im geschmolzenen Zustand bereitgestellt wird und vorzugsweise die kombinierte Gesamtmenge an Lactid(en) und Glycolid in der besagten Mischung mindestens 70,0 Gew.-%, bezogen auf das Gesamtgewicht der Mischung, ist,
b) Ermitteln des Kristallisationspunkts der Mischung,
c) Ermitteln einer gewünschten Menge an Lactid, die aus der besagten Mischung zurückzugewinnen ist,
d) Auswählen einer geeigneten Kristallisationstemperatur (T_{c}) basierend auf der gewünschten Menge an Lactid, die zurückzugewinnen ist, wie in Schritt c) ermittelt,
e) Unterziehen der besagten Mischung einer Schmelzkristallisation bei der besagten Temperatur T_{c}, vorzugsweise bei einer Kristallisationstemperatur (T_{c}) zwischen 30 °C und 95 °C oder zwischen 50 °C und 95 °C oder zwischen 60 °C und 90 °C, wodurch eine Mutterflüssigkeit und kristallisiertes Lactid zurückgewonnen werden, und
f) Zurückgewinnen des besagten kristallisierten Lactids,
g) optional weiter Aufreinigen des in Schritt f) zurückgewonnenen kristallisierten Lactids mittels Schmelzens des besagten kristallisierten Lactids zu einem geschmolzenen Produkt und Zuführens des geschmolzenen Produkts als eine neue Mischung in Schritt a), wodurch aufgereinigtes kristallisiertes Lactid und eine neue Mutterflüssigkeit aus dem zurückgewonnenen kristallisierten Lactid zurückgewonnen werden, und
h) optional Zuführen von Mutterflüssigkeit(n), die in irgendeinem der vorherigen Schritte gewonnen wurden, als eine neue Mischung in Schritt a), wodurch zusätzliche Mengen an kristallisiertem Lactid und eine neue Mutterflüssigkeit zurückgewonnen werden.

10. Verfahren gemäß Anspruch 9, wobei das kristallisierte Lactid eine Menge (in Gew.-%) an Glycolid aufweist, die weniger als das 0,5-Fache, beispielsweise weniger als das 0,2-Fache, der Menge (in Gew.-%) an Glycolid in der Mischung ist.

11. Verfahren gemäß Anspruch 9 oder 10, wobei der Aufreinigungsfaktor für Glykolid des kristallisierten Lactids im Bereich von 2 bis 20 ist.

12. Verfahren gemäß irgendeinem der Ansprüche 1 bis 8, wobei Glykolid aus einer Mischung abgetrennt wird, die Lactid(e) und Glykolid aufweist, wobei das Verfahren die Schritte aufweist:
a) Bereitstellen einer Mischung, die Lactid(e) und Glykolid aufweist, wobei die besagte Mischung im geschmolzenen Zustand bereitgestellt wird und vorzugsweise wobei die kombinierte Gesamtmenge an Lactid(en) und Glykolid in der besagten Mischung mindestens 70,0 Gew.-%, bezogen auf das Gesamtgewicht der Mischung, ist,
b) Ermitteln des Kristallisationspunkts der besagten Mischung,
c) Ermitteln einer gewünschten Menge an Glykolid, die aus der besagten Mischung zurückzugewinnen ist,
d) Auswählen einer geeigneten Kristallisationstemperatur (T_{c}) basierend auf der gewünschten Menge an Glykolid, die zurückzugewinnen ist, wie in Schritt c) ermittelt,
e) Unterziehen der besagten Mischung einer Schmelzkristallisation bei der besagten Temperatur T_{c}, und vorzugsweise einer Kristallisationstemperatur (T_{c}) zwischen 50 °C und 80 °C, beispielsweise zwischen 60 °C und 80 °C, wodurch eine Mutterflüssigkeit und ein kristallisiertes Glykolid, das Glykolidkristalle aufweist, erlangt werden, und
f) Zurückgewinnen des kristallisierten Glykolids,
g) optional weiter Aufreinigen des in Schritt f) zurückgewonnenen kristallisierten Glykolids mittels Schmelzens des kristallisierten Glykolids zu einem geschmolzenen Produkt und Zuführen des geschmolzenen Produkts als eine neue Mischung in Schritt a), wodurch aufgereinigtes kristallisiertes Glykolid und eine neue Mutterflüssigkeit aus dem besagten zurückgewonnenen kristallisierten Glykolid zurückgewonnen werden, und
h) optional Zuführen von Mutterflüssigkeit(n), die in irgendeinem der vorherigen Schritte erlangt wurden, als eine neue Mischung in Schritt a), wodurch zusätzliche Mengen an kristallisiertem Glykolid und eine neue Mutterflüssigkeit zurückgewonnen werden.

13. Verfahren gemäß irgendeinem der Ansprüche 1 bis 8 und 12, wobei das kristallisierte Glykolid eine Menge (in Gew.-%) an Lactid(en) aufweist, die weniger als das 0,5-Fache, beispielsweise weniger als das 0,2-Fache, der Menge (in Gew.-%) an Lactid(en) in der Mischung ist.

14. Verfahren gemäß irgendeinem der Ansprüche 1 bis 8 und 12 bis 13, wobei der Aufreinigungsfaktor für Lactid des kristallisierten Glycolids im Bereich von 2 bis 20 ist.

15. Verfahren gemäß irgendeinem der vorstehenden Ansprüche, wobei die in Schritt a) bereitgestellte Mischung aus Lactid(en) und Glycolid als ein Nebenprodukt bei einem Verfahren zum Herstellen von Poly(lactid-co-glykolsäure) (PLGA) mittels Ringöffnungscopolymerisation von Lactid(en) und Glycolid erlangt wird.

16. Verfahren gemäß irgendeinem der vorstehenden Ansprüche, wobei die in Schritt a) bereitgestellte Mischung, die Lactid(e) und Glykolid aufweist, mittels thermischer Depolymerisation von PLGA erlangt wird.

17. Verfahren gemäß irgendeinem der vorstehenden Ansprüche, wobei die in Schritt a) bereitgestellte Mischung, die Lactid(en) und Glycolid aufweist, aus einer Mischung, die Milchsäure und Glykolsäure aufweist, hergestellt wird und vorzugsweise hergestellt wird mittels der Schritte:
a1) Bereitstellen einer Mischung, die Milchsäure und Glykolsäure aufweist,
a2) Unterziehen der besagten Mischung, die Milchsäure und Glykolsäure aufweist, einer Polykondensation unter Bedingungen, die zum Bilden von Milchsäure/Glykolsäure-Co-Oligomeren effektiv sind, und
a3) Depolymerisieren der in Schritt a2) gebildeten Milchsäure/Glykolsäure-Co-Oligomere, um eine Mischung zu bilden, die Lactid(e) und Glykolid aufweist.

## Revendications

1. Procédé de séparation d'un mélange comprenant un ou plusieurs lactides et un glycolide, dans lequel ledit procédé comprend les étapes suivantes :
a) la fourniture d'un mélange comprenant un ou plusieurs lactides et un glycolide, dans lequel ledit mélange est fourni à l'état fondu, et de préférence dans lequel la quantité totale combinée de lactide(s) et de glycolide dans ledit mélange est d'au moins 70,0 % en poids, sur la base du poids total du mélange,
b) la détermination du point de cristallisation dudit mélange,
c) la détermination d'une quantité souhaitée de lactide ou de glycolide à récupérer à partir dudit mélange,
d) la sélection d'une température de cristallisation (T_{c}) appropriée sur la base de la quantité souhaitée de lactide ou de glycolide à récupérer telle que déterminée à l'étape c),
e) la soumission dudit mélange à une cristallisation par fusion à ladite température T_{c}, pour ainsi obtenir une solution mère et un produit cristallisé, dans lequel ledit produit cristallisé comprend du lactide cristallisé ou du glycolide cristallisé, et
f) la récupération dudit produit cristallisé.

2. Procédé selon la revendication 1, dans lequel le point de cristallisation du mélange est déterminé par établissement d'un diagramme de phase du glycolide et du lactide qui est le lactide prédominant dans ledit mélange.

3. Procédé selon l'une quelconque des revendications précédentes, dans lequel la température de cristallisation (T_{c}) est comprise dans une plage de 30 à 95 °C, ou de 50 à 95 °C, ou de 50 à 80 °C, ou de 60 à 90 °C.

4. Procédé selon l'une quelconque des revendications précédentes, dans lequel le produit cristallisé récupéré à l'étape f) est en outre purifié par fusion dudit produit cristallisé en un produit fondu et par fourniture du produit fondu en tant que nouveau mélange à l'étape a) du procédé de séparation de l'une quelconque des revendications précédentes, pour ainsi récupérer un produit cristallisé purifié et une nouvelle solution mère à partir dudit produit cristallisé récupéré.

5. Procédé selon l'une quelconque des revendications précédentes, dans lequel la solution mère obtenue à l'étape e) est fournie en tant que nouveau mélange à l'étape a) du procédé de séparation selon l'une quelconque des revendications précédentes, pour ainsi récupérer des quantités supplémentaires de produit cristallisé et une nouvelle solution mère.

6. Procédé selon l'une quelconque des revendications précédentes, dans lequel au moins 50 %, ou au moins 60 %, ou au moins 70 %, ou au moins 75 %, ou au moins 80 %, ou au moins 85 %, ou au moins 90 % de la quantité de lactide ou de glycolide comprise dans le mélange est récupérée sous forme de produit cristallisé.

7. Procédé selon l'une quelconque des revendications précédentes, dans lequel la cristallisation par fusion comprend
- au moins une cristallisation en couche, et de préférence ladite cristallisation en couche comprend au moins une cristallisation en film tombant et/ou au moins une cristallisation statique, et/ou
- au moins une cristallisation en suspension.

8. Procédé selon l'une quelconque des revendications précédentes, dans lequel le produit cristallisé, et facultativement le produit cristallisé purifié, sont récupérés par filtration, centrifugation ou un autre moyen de séparation solide-liquide, ou toute combinaison de ceux-ci.

9. Procédé selon l'une quelconque des revendications précédentes, dans lequel un lactide est séparé d'un mélange comprenant un ou plusieurs lactides et un glycolide, et dans lequel ledit procédé comprend les étapes suivantes :
a) la fourniture d'un mélange comprenant un ou plusieurs lactides et un glycolide, dans lequel ledit mélange est fourni à l'état fondu, et de préférence dans lequel la quantité totale combinée de lactide(s) et de glycolide dans ledit mélange est d'au moins 70,0 % en poids, sur la base du poids total du mélange,
b) la détermination du point de cristallisation dudit mélange,
c) la détermination d'une quantité souhaitée de lactide à récupérer à partir dudit mélange,
d) la sélection d'une température de cristallisation (T_{c}) appropriée sur la base de la quantité souhaitée de lactide à récupérer telle que déterminée à l'étape c),
e) la soumission dudit mélange à une cristallisation par fusion à ladite température T_{c}, de préférence une température de cristallisation (T_{c}) comprise entre 30 °C et 95 °C, ou entre 50 °C et 95 °C, ou entre 60 °C et 90 °C, pour ainsi obtenir une solution mère et un lactide cristallisé ; et
f) la récupération dudit lactide cristallisé,
g) la purification supplémentaire facultative du lactide cristallisé récupéré à l'étape f) par fusion dudit lactide cristallisé en un produit fondu et par fourniture du produit fondu en tant que nouveau mélange à l'étape a), pour ainsi récupérer un lactide cristallisé purifié et une nouvelle solution mère à partir dudit lactide cristallisé récupéré, et
h) la fourniture facultative de la ou des solutions mères obtenues dans l'une quelconque des étapes précédentes, en tant que nouveau mélange à l'étape a), pour ainsi récupérer des quantités supplémentaires de lactide cristallisé et une nouvelle solution mère.

10. Procédé selon la revendication 9, dans lequel le lactide cristallisé comprend une quantité (en % en poids) de glycolide qui est inférieure à 0,5 fois, telle qu'inférieure à 0,2 fois, la quantité (en % en poids) de glycolide dans le mélange.

11. Procédé selon la revendication 9 ou 10, dans lequel le facteur de purification pour le glycolide du lactide cristallisé est dans la plage de 2 à 20.

12. Procédé selon l'une quelconque des revendications 1 à 8, dans lequel un glycolide est séparé d'un mélange comprenant un ou plusieurs lactides et un glycolide, dans lequel ledit procédé comprend les étapes suivantes :
a) la fourniture d'un mélange comprenant un ou plusieurs lactides et un glycolide, dans lequel ledit mélange est fourni à l'état fondu, et de préférence dans lequel la quantité totale combinée de lactide(s) et de glycolide dans ledit mélange est d'au moins 70,0 % en poids, sur la base du poids total du mélange,
b) la détermination du point de cristallisation dudit mélange,
c) la détermination d'une quantité souhaitée de glycolide à récupérer à partir dudit mélange,
d) la sélection d'une température de cristallisation (T_{c}) appropriée sur la base de la quantité souhaitée de glycolide à récupérer telle que déterminée à l'étape c),
e) la soumission dudit mélange à une cristallisation par fusion à ladite température T_{c}, et de préférence à une température de cristallisation (T_{c}) comprise entre 50 °C et 80 °C, telle qu'entre 60 °C et 80 °C, pour ainsi obtenir une solution mère et un glycolide cristallisé comprenant des cristaux de glycolide ; et
f) la récupération dudit glycolide cristallisé,
g) la purification supplémentaire facultative du glycolide cristallisé récupéré à l'étape f) par fusion dudit glycolide cristallisé en un produit fondu et par fourniture du produit fondu en tant que nouveau mélange à l'étape a), pour ainsi récupérer un glycolide cristallisé purifié et une nouvelle solution mère à partir dudit glycolide cristallisé récupéré, et
h) la fourniture facultative de la ou des solutions mères obtenues dans l'une quelconque des étapes précédentes, en tant que nouveau mélange à l'étape a), pour ainsi récupérer des quantités supplémentaires de glycolide cristallisé et une nouvelle solution mère.

13. Procédé selon l'une quelconque des revendications 1 à 8 et 12, dans lequel le glycolide cristallisé comprend une quantité (en % en poids) de lactide(s) qui est inférieure à 0,5 fois, telle qu'inférieure à 0,2 fois, la quantité (en % en poids) de lactide(s) dans le mélange.

14. Procédé selon l'une quelconque des revendications 1 à 8 et des revendications 12 à 13, dans lequel le facteur de purification pour le lactide du glycolide cristallisé est dans la plage de 2 à 20.

15. Procédé selon l'une quelconque des revendications précédentes, dans lequel le mélange comprenant un ou plusieurs lactides et un glycolide fourni à l'étape a) est obtenu en tant que sous-produit dans un procédé de préparation de poly(acide lactique-co-glycolique) (PLGA) par copolymérisation par ouverture de cycle du ou des lactides et du glycolide.

16. Procédé selon l'une quelconque des revendications précédentes, dans lequel le mélange comprenant un ou plusieurs lactides et un glycolide fourni à l'étape a) est obtenu par dépolymérisation thermique du PLGA.

17. Procédé selon l'une quelconque des revendications précédentes, dans lequel le mélange comprenant un ou plusieurs lactides et un glycolide fourni à l'étape a) est préparé à partir d'un mélange comprenant de l'acide lactique et de l'acide glycolique, et est de préférence préparé par les étapes suivantes :
a1) la fourniture d'un mélange comprenant de l'acide lactique et de l'acide glycolique ;
a2) la soumission dudit mélange comprenant de l'acide lactique et de l'acide glycolique à une polycondensation dans des conditions efficaces pour former des co-oligomères d'acide lactique/acide glycolique ; et,
a3) la dépolymérisation des co-oligomères d'acide lactique/acide glycolique formés à l'étape a2) pour former un mélange comprenant un ou plusieurs lactides et un glycolide.
